# EUROPEAN PATENT APPLICATION

(11) **EP 2 275 150 A2**
(43) Date of publication of application: **19.01.2011**
(21) Application number: 10251273.8
(22) Date of filing: 16.07.2010
(51) Int. Cl.: A61L 17/14, A61L 27/34

(54) **Method for coating a medical device**

(30) Priority: 17.07.2009 US 226321 P; 12.05.2010 US 778445
(71) Applicant: Tyco Healthcare Group, LP, New Haven CT 06511 (US)
(72) Inventor: Stopek, Joshua, Guilford, CT 06437 (US)
(74) Representative: Soames, Candida Jane

(57) **Abstract**

The present disclosure provides coatings for medical devices, methods for applying such coatings, and medical devices possessing such coatings.

## Description

### CROSS-REFERENCE TO RELATED APPLICATION

The present application claims the benefit of and priority to U.S. Provisional Application Ser. No. 61/226,321, filed on July 17, 2009, the entire disclosure of which is incorporated herein by reference.

### BACKGROUND

### Technical Field

The present disclosure relates to a method for coating a medical device and, more particularly, to a two-step method for coating a medical device, such as a suture.

### Background of Related Art

It is well known in the art that methods for coating a medical device, such as a suture, may be utilized to enhance certain surface properties of a device, such as ease of sliding a knot into place, also known as knot-repositioning, lubricity, bacterial adhesion prevention, cell and protein adhesion, drug delivery, and protein and DNA delivery and immobilization.

U.S. Patent No. 5,312,437 discloses an absorbable suture coating composition including a product obtained by reacting a mixture of poly(oxypropylene)glycol and a copolymer of lactide and glycolide.

U.S. Patent No. 5,425,949 discloses a bioabsorbable copolymer obtained by polymerizing a major amount of epsilon-caprolactone and a minor amount of at least one other copolymerizable monomer in the presence of a polyhydric alcohol initiator. The copolymer can be used as a suture coating.

Notwithstanding these known methods, it would be advantageous to provide methods for coating medical devices that prevent bacterial adhesion, colonization and device-associated infection, as well as providing useful properties such as lubricity and drug delivery capabilities.

### SUMMARY

Methods are described wherein coatings are applied to medical devices. In embodiments, the methods include pre-coating a medical device with a composition containing at least one isocyanate-terminated polymer and contacting the pre-coated medical device with at least one polyamine compound to crosslink the at least one isocyanate-terminated polymer.

Medical devices possessing such coatings are also provided.

### DETAILED DESCRIPTION

The present methods can be used to coat various medical devices. Some examples of medical devices which may be coated in accordance with the present disclosure include, but are not limited to, sutures, staples, meshes, patches, slings, stents, catheters, endotracheal tubes, grafts, clips, pins, screws, rivets, tacks, bone plates, drug delivery devices, adhesives, sealants, wound dressings, woven devices, non-woven devices, braided devices, adhesion barriers, tissue scaffolds, and other implants. In certain embodiments, the medical device may be formed from one or more filaments. The filaments can be knitted, braided, woven or non-woven. In one embodiment, the medical device may be a suture.

The medical device can be formed from any sterilizable material that has suitable physical properties for the intended use of the medical device. The medical device can be bioabsorbable or non-bioabsorbable. Some specific examples of suitable absorbable materials which may be utilized to form the medical device include trimethylene carbonate, caprolactone, dioxanone, glycolic acid, lactic acid, glycolide, lactide, homopolymers thereof, copolymers thereof, and combinations thereof. Some specific examples of suitable non-absorbable materials which may be utilized to form the medical device include polyolefins such as polyethylene, polypropylene, copolymers of polyethylene and polypropylene, and blends of polyethylene and polypropylene.

The methods for coating a medical device disclosed herein include a two-step process. The first step includes pre-coating a medical device with a composition containing at least one isocyanate-terminated polymer. The second step includes contacting the pre-coated medical device with at least one polyamine compound to crosslink the at least one isocyanate-terminated polymer.

The present methods can utilize any isocyanate-terminated polymer within the purview of one skilled in the art to form the pre-coated medical device. Some examples include, but are not limited to, isocyanate-terminated polymers such as polyethers, polyesters or poly(ether-ester) blocks. Suitable polyethers which may be utilized are within the purview of those skilled in the art and include, for example, polyalkylene oxides such as polyethylene oxide, polypropylene oxide, polyethylene glycol, polypropylene glycol, polybutylene glycol, polytetramethylene glycol, polyhexamethylene glycol, copolymers thereof, for example, poly(ethylene glycol-co-propylene glycol), and combinations thereof. Other polyalkylene oxides which may be utilized include co-polyethylene oxide block or random copolymers, and poloxamers such as polyethylene oxide (PEO) copolymers with polypropylene oxide (PPO) such as the triblock PEO - PPO copolymers commercially available as PLURONICS® from BASF Corporation (Mt. Olive, NJ).

In embodiments, a suitable polyalkylene oxide includes a polyethylene oxide, such as a polyethylene glycol(PEG). As used herein, polyethylene glycol generally refers to a polymer with a molecular weight of less than 50,000 g/mol, while polyethylene oxide is used for higher molecular weights. PEGs provide excellent water retention, flexibility and viscosity in the biocompatible synthetic macromer composition.

In embodiments, a polyalkylene oxide having a molecular weight greater than about 500 may be utilized, in embodiments a molecular weight from about 500 to about 1000 may be utilized. For example, in one embodiment, a polyethylene glycol having a molecular weight of about 600 (PEG 600) may be utilized.

In embodiments, the isocyanate-terminated polymer may be a polyethylene glycol at a concentration from about 1% to about 90 %, in embodiments from about 5% to about 80 %. It is envisioned that increasing the polyethylene glycol concentration of the coating may increase the repulsive force of the surface of the medical device, thereby improving the molecular mobility and hydrophilicity of the coating and reducing cell and protein adhesion.

Suitable polyesters which may be terminated with isocyanate and utilized as a component of a coating of the present disclosure are within the purview of those skilled in the art and include, for example, trimethylene carbonate, ε-caprolactone, p-dioxanone, glycolide, lactide, 1,5-dioxepan-2-one, polybutylene adipate, polyethylene adipate, polyethylene terephthalate, and combinations thereof. In embodiments the polyester may be lactide, glycolide, ε-caprolactone, and/or combinations thereof. For example, in embodiments, the polyester may be a glycolide/caprolactone copolymer.

In addition, the isocyanate-terminated polymer may include a poly(ether-ester) block. Any suitable poly(ether-ester) block within the purview of those skilled in the art may be utilized. Some examples include, but are not limited too, polyethylene glycol-polycaprolactone, polyethylene glycol-polylactide, polyethylene glycol-polyglycolide, polyethylene glycol-glycolide/caprolactone copolymer, and various combinations of the individual polyethers and polyesters described herein. Additional examples of poly(ether-ester) blocks are disclosed in U.S. Patent No. 5,578,662 and U.S. Patent Application No. 2003/0135238, the entire disclosures of each of which are incorporated by reference herein.

As noted above, the isocyanate-terminated polymers of the present disclosure include polymers as described herein that are end-capped with at least one isocyanate. Suitable isocyanates which may be utilized to end-cap a polymer are within the purview of those skilled in the art and include aromatic, aliphatic and alicyclic isocyanates. Examples include, but are not limited to, aromatic diisocyanates such as 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenylisocyanate), tetramethylxylylene diisocyanate, and combinations thereof; aliphatic diisocyanates such as tetramethylene diisocyanate, hexamethylene diisocyanate, dimethyl diisocyanate, lysine diisocyanate, 2-methylpentane-1,5-diisocyanate, 3-methylpentane-1,5-diisocyanate, 2,2,4-trimethylhexamethylene diisocyanate, and combinations thereof; and alicyclic diisocyanates such as isophorone diisocyanate, cyclohexane diisocyanate, hydrogenated xylylene diisocyanate, hydrogenated diphenylmethane diisocyanate, hydrogenated trimethylxylylene diisocyanate, 2,4,6-trimethyl 1,3-phenylene diisocyanate, commercially available isocyanates including those sold under the name DESMODURS^{®} from Bayer Material Science, and combinations thereof.

Methods for endcapping the polymer with a diisocyanate are within the purview of those skilled in the art. In some embodiments, the polymer may be combined with a suitable diisocyanate, such as a toluene diisocyanate, and heated to a suitable temperature from about 55° C to about 75° C, in embodiments from about 60° to about 70° C, in embodiments about 65° C. The amount of diisocyanate employed can be from about 2 to about 8 moles of diisocyanate per mole of polymer. Suitable reaction times can be from about 15 minutes to about 72 hours or more. In some embodiments the resulting diisocyanate-functional compound may then be obtained by hot extraction with petroleum ether.

Isocyanate-terminated polymers for use in forming coatings in accordance with the present disclosure may have a molecular weight of from about 600 to about 15,000 g/mol, and in embodiments from about 1,500 to about 5,000 g/mol.

Once obtained, the isocyanate-terminated polymer may be applied to a medical device. Methods for applying the isocyanate-terminated polymer are within the purview of those skilled in the art. In embodiments the isocyanate-terminated polymer may be placed in a suitable solvent and the solution applied to a medical device by appropriate methods including, but not limited to, dipping, brushing, spraying, and the like. Suitable solvents which may be utilized include those within the purview of those skilled in the art for use in coating medical devices such as alcohols including methanol, ethanol, and propanol; chlorinated hydrocarbons including methylene chloride, chloroform, and 1,2-dichloro-ethane, aliphatic hydrocarbons including hexane, heptene, ethyl acetate,

combinations thereof, and the like. As would be readily apparent to one skilled in the art, the solvent utilized should not degrade the medical device in a negative fashion.

In some embodiments, once the isocyanate-terminated polymer in solution has been applied to a medical device, the solvent may be removed, in embodiments by heating, thereby leaving the isocyanate-terminated polymer on the surface of the medical device. In other embodiments the isocyanate-terminated polymer in solution may be left on the medical device forming a wetted medical device.

The present methods also include the second step of contacting the pre-coated medical device with a composition containing at least one polyamine compound to crosslink the isocyanate-terminated polymer. Suitable polyamine compounds which may be utilized are within the purview of those skilled in the art and include, for example, polylysine, trilysine, chitosan, diamines, including hexamethylene diamine, ethylenediamine, N-ethylethylenediamine, N,N'-diethylethylenediamine, spermine, spermidine, and alkanolamines. Examples of alkanolamines which may be utilized include dihydric and trihydric alkanolamines, such as ethanolamine and N-ethylethanolamine, triethylenediamine, N-methylmorpholine, pentamethyl diethylenetriamine, dimethylcyclohexylamine, tetramethylethylenediamine, 1 - methyl-4-dimethylaminoethyl-piperazine, 3-methoxy-N-dimethyl-propylamine, N-ethylmorpholine, diethylethanolamine, N-cocomorpholine, N,N-dimethyl-N',N'-dimethylisopropylpropylene diamine, N,N-diethyl-3-diethyl aminopropylamine and dimethyl-benzyl amine. Combinations of the foregoing polyamine compounds may be utilized in some embodiments. In some embodiments, the polyamine compound may include polylysine, chitosan, hexamethylene diamine, spermine and combinations thereof.

Free amine groups of the polyamine will react with free isocyanate groups on the isocyanate-terminated polymer already applied to a medical device, thereby forming a coating of the present disclosure.

In certain embodiments, the polyamine may be present in a coating in an amount of from about 0.1 % to about 90 % by weight of the coating, in embodiments of from about 5 % to about 80 % by weight of the coating, in other embodiments of from about 10 % to about 75 % by weight of the coating.

Polyamines utilized in forming coatings in accordance with the present disclosure may have a molecular weight of from about 500 to about 100,000 g/mol, and in embodiments from about 1,000 to about 50,000 g/mol.

It is envisioned that the polyamine compounds can be applied to a medical device by any suitable process, e.g., passing the suture through a solution of the polyamine, or past a brush or other coating solution applicator, or past one or more spray nozzles dispensing the polyamine, optionally in solution, or dipped directly into the polyamine compound. Where the polyamine is in solution, any suitable solvent for medical devices may be utilized.

It is also envisioned that the coated medical device can be subsequently passed through or held in a drying oven for a sufficient amount of time to cure the coated device and enhance binding of the polyamine to the isocyanate-terminated polymer.

In embodiments, the methods described herein produce a smooth, laminar hydrophilic polymer coating on a medical device. In other embodiments, the methods described herein may produce a porous hydrophilic polymer coating on a medical device.

It is envisioned that a porous hydrophilic polymer coating may be formed on a medical device by combining a pore-forming agent with the isocyanate-terminated polymer prior to coating the medical device. Pore-forming agents may generally be added as particulates and include water-soluble compounds such as inorganic salts and sugars. Suitable pore-forming agents include salt crystals such as sodium chloride, sodiium citrate, sodium tartrate, sodium carbonate, sodium bicarbonate and potassium chloride, proteins such as gelatin, collagen and agarose, starches, polysaccharides such as alginate, other similar polymers, and combinations thereof. These pore-forming agents may be removed, in embodiments, by leaching. It is also possible to use particulate pore-forming agents which are leachable by organic solvents, where the solvent does not adversely affect the polymer. The diameters of the particles utilized as the pore-forming agents may be from about 10 nanometers to about 500 microns. They may also be lyophilizable. Pore-forming agents can be included in an amount of from about 0.1 % to about 75% by weight of the coating, in embodiments from 5% to about 50% by weight of the coating, to increase pore formation.

It is further envisioned that the polyamine compound may also be combined with additional ingredients, including one or more bioactive agents. The term "bioactive agent", as used herein, is used in its broadest sense and includes any substance or mixture of substances that have clinical use. Consequently, bioactive agents may or may not have pharmacological activity per se, e.g., a dye, or fragrance. Alternatively, a bioactive agent could be any agent which provides a therapeutic or prophylactic effect, a compound that affects or participates in tissue growth, cell growth or cell differentiation, a compound that may be able to invoke a biological action such as an immune response, or could play any other role in one or more biological processes.

Examples of classes of bioactive agents which may be utilized in accordance with the present disclosure include antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, enzymes, polymer-drugs, bioactive polymers, and combinations thereof. Some non-limiting examples of bioactive polymers include polymers which include phospholipids, hydroxymates, extracellular matrix proteins/peptides and furanones.

Suitable antimicrobial agents which may be included as a bioactive agent in the bioactive coating of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a bioactive agent in the bioactive coating of the present disclosure.

Other bioactive agents which may be included in accordance with the present disclosure include local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g. oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable bioactive agents which may be included in coatings of the present disclosure include viruses and cells, peptides, polypeptides and proteins, analogs, muteins, and active fragments thereof, such as immunoglobulins, antibodies, cytokines (e.g. lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, gonadotropins (e.g., FSH, LH, CG, etc.), hormones and hormone analogs (e.g., growth hormone), vaccines (e.g., tumoral, bacterial and viral antigens), somatostatin, antigens, blood coagulation factors, growth factors (e.g., nerve growth factor, insulin-like growth factor), protein inhibitors, protein antagonists, protein agonists, nucleic acids such as antisense molecules, DNA and RNA, oligonucleotides, polynucleotides, and ribozymes.

It is envisioned that a single bioactive agent may be utilized in the coating composition or, in alternate embodiments, any combination of bioactive agents may be utilized in the coating composition applied in accordance with the present disclosure.

The following Examples are being submitted to illustrate embodiments of the present disclosure. These Examples are intended to be illustrative only and are not intended to limit the scope of the present disclosure. Also, parts and percentages are by weight unless otherwise indicated.

### EXAMPLE 1

A surgical suture was pre-coated with a copolymer including 25% lactide, 70% polyethylene glycol (PEG) and 5% hexamethylene diisocyanate (HMDI), in methylene chloride. The copolymer had a viscosity of about 100 centipoise suitable for fiber wetting or spreading. The pre-coated surgical suture was subsequently contacted with a 10% solution of polylysine in water to react with the available isocyanate functionalities of the isocyanate-terminated polymer coating used to pre-coat the suture. A smooth, laminar, hydrophilic polymer coating formed on the medical device.

### EXAMPLE 2

A surgical suture was pre-coated with a copolymer including 25% lactide, 70% polyethylene glycol (PEG), and 5% hexamethylene diisocyanate (HMDI) in methylene chloride. The pre-coated suture was subsequently contacted with a solution including 10% polylysine and 20% sodium bicarbonate in water to react with the available isocyanate functionalities of the isocyanate-terminated polymer used to coat the suture. The coated suture was processed further by washing the coated suture in water to remove the pore-forming agent thereby resulting in a porous, hydrophilic polymer coating.

While the above description contains many specifics, these specifics should not be construed as limitations on the scope of the disclosure, but merely as exemplifications of embodiments thereof. Those skilled in the art will envision many other possibilities within the scope and spirit of the disclosure as defined by the claims appended hereto.

## Claims

1. A method for coating a medical device comprising:
pre-coating a medical device with a composition comprising at least one isocyanate-terminated polymer; and
contacting the pre-coated medical device with at least one polyamine compound to crosslink the at least one isocyanate-terminated polymer.

2. The method of claim 1 wherein the at least one isocyanate-terminated polymer comprises a polymer selected from the group consisting of polyethers, polyesters, poly(ether-ester) blocks, and combinations thereof, preferably wherein the polyethers are selected from the group consisting of polyethylene glycol, polypropylene glycol, poly(ethylene glycol-co-propylene glycol), polybutylene glycol, polytetramethylene glycol, polyhexamethylene glycol, and combinations thereof and/or wherein the polyesters are selected from the group consisting of lactide, glycolide, ε-caprolactone, and combinations thereof.

3. The method of claim 1 or claim 2 wherein the at least one polyamine compound is selected from the group consisting of polylysine, trilysine, chitosan, hexamethylene diamine, ethylenediamine, N-ethylethylenediamine, N,N'-diethylethylenediamine, spermine, spermidine, and combinations thereof or the method of claim 1 wherein the at least one polyamine compound is selected from the group consisting of ethanolamine, N-ethylethanolamine, triethylenediamine, N-methylmorpholine, pentamethyl diethylenetriamine, dimethylcyclohexylamine, tetramethylethylenediamine, 1 -methyl-4-dimethylaminoethyl-piperazine, 3-methoxy-N-dimethyl-propylamine, N-ethylmorpholine, diethylethanolamine, N-cocomorpholine, N,N-dimethyl-N',N'-dimethylisopropyl-propylene diamine, N,N-diethyl-3-diethyl aminopropylamine, dimethyl-benzyl amine, and combinations thereof.

4. The method of claim 1 or claim 2 wherein the pre-coating composition further comprises a pore-forming agent.

5. The method of claim 4 wherein the pore-forming agent is selected from the group consisting of salts, proteins, starches, polysaccharides, and combinations thereof.

6. The method of claim 4 wherein the pore-forming agent is selected from the group consisting of sodium chloride, sodium citrate, sodium tartrate, potassium chloride, gelatin, collagen, agarose, alginate, sodium carbonate, sodium bicarbonate, and combinations thereof.

7. The method of any preceding claim wherein the crosslinking composition further comprises a bioactive agent, preferably wherein the bioactive agent is selected from the group consisting of antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, enzymes, and combinations thereof.

8. The method of claim 7 wherein the bioactive agent is selected from the group consisting of viruses, peptides, polypeptides, proteins, protein inhibitors, protein antagonists, protein agonists, nucleic acids, oligonucleotides, polynucleotides, ribozymes, and combinations thereof.

9. A medical device coated using the method of any preceding claim.

10. The medical device of claim 9 wherein the medical device is selected from the group consisting of sutures, staples, meshes, patches, slings, stents, catheters, endotracheal tubes, grafts, clips, pins, screws, rivets, tacks, bone plates, drug delivery devices, adhesives, sealants, wound dressings, adhesion barriers, tissue scaffolds, and implants.

11. The medical device of claim 10 wherein the medical device is a suture.

12. A method for coating a suture comprising:
applying a pre-coating composition to a suture to provide a pre-coated suture, the pre-coating composition comprising at least one isocyanate-terminated polymer; and
contacting the pre-coated suture with a crosslinking composition comprising at least one polyamine compound.

13. The method of claim 12 wherein the isocyanate-terminated polymer comprises a bioabsorbable polyether-ester comprising a polyalkylene oxide and at least one monomer selected from the group consisting of lactide, glycolide, ε-caprolactone, trimethylene carbonate, p-dioxanone and 1,5-dioxepan-2-one.

14. The method of claim 12 or 13 wherein the bioabsorbable polyether-ester is endcapped with a diisocyanate selected from the group consisting of 2,4-toluene diisocyanate, 2,6-toluene diisocyanate, 2,2'-diphenylmethane diisocyanate, 2,4'-diphenylmethane diisocyanate, 4,4'-diphenylmethane diisocyanate, diphenyldimethylmethane diisocyanate, dibenzyl diisocyanate, naphthylene diisocyanate, phenylene diisocyanate, xylylene diisocyanate, 4,4'-oxybis(phenylisocyanate), tetramethylxylylene diisocyanate, tetramethylene diisocyanate, hexamethylene diisocyanate, dimethyl diisocyanate, lysine diisocyanate, 2-methylpentane-1,5-diisocyanate, 3-methylpentane-1,5-diisocyanate, 2,2,4-trimethylhexamethylene diisocyanate, isophorone diisocyanate, cyclohexane diisocyanate, hydrogenated xylylene diisocyanate, hydrogenated diphenylmethane diisocyanate, hydrogenated trimethylxylylene diisocyanate, 2,4,6-trimethyl 1,3-phenylene diisocyanate, and combinations thereof.

15. The method of any of claims 12 to 14 wherein the crosslinking composition comprises at least one polyamine selected from the group consisting of polylysine, chitosan, hexamethylene diamine, ethylenediamine, N-ethylethylenediamine, N,N'-diethylethylenediamine, spermine, spermidine, ethanolamine and N-ethylethanolamine, triethylenediamine, N-methylmorpholine, pentamethyl diethylenetriamine, dimethylcyclohexylamine, tetramethylethylenediamine, 1-methyl-4-dimethylaminoethyl-piperazine, 3-methoxy-N-dimethyl-propylamine, N-ethylmorpholine, diethylethanolamine, N-cocomorpholine, N,N-dimethyl-N',N'-dimethylisopropyl-propylene diamine, N,N-diethyl-3-diethyl aminopropylamine, dimethyl-benzyl amine and combinations thereof.

16. The method of any of claims 12 to 15 wherein the crosslinking composition further comprises a pore-forming agent.

17. The method of claim 16 wherein the pore-forming agent is selected from the group consisting of salts, proteins, starches, polysaccharides, and combinations thereof, preferably wherein the pore-forming agent is selected from the group consisting of sodium chloride, sodium citrate, sodium tartrate, potassium chloride, gelatin, collagen, agarose, alginate, sodium carbonate, sodium bicarbonate, and combinations thereof.
